# FASCICULE DE BREVET EUROPEEN

(11) **EP 3 558 180 B1**
(45) Date de publication et mention de la délivrance du brevet: **03.04.2024**
(21) Numéro de dépôt: 17817004.9
(22) Date de dépôt: 07.12.2017
(51) Int. Cl.: A61F 9/08

(54) **SYSTEME DE SUBSTITUTION SENSORIELLE PAR STIMULATION TACTILE ASYNCHRONE**
SYSTEM ZUR SENSORISCHEN SUBSTITUTION DURCH ASYNCHRONE TAKTILE STIMULATION
SYSTEM FOR SENSORY SUBSTITUTION BY ASYNCHRONOUS TACTILE STIMULATION

(30) Priorité: 20.12.2016 FR 1662851
(43) Date de publication de la demande: 30.10.2019
(73) Titulaire: SORBONNE UNIVERSITE, 75006 Paris (FR); Centre National de la Recherche Scientifique - CNRS, 75016 Paris (FR); Institut National de la Santé et de la Recherche Médicale (INSERM), 75013 Paris (FR)
(72) Inventeur: CHENEGROS, Guillaume, 78190 Trappes (FR); BENOSMAN, Ryad, 93500 Pantin (FR); ARTH, Kevin, 75019 Paris (FR); IENG, Sio-Hoi, 93100 Montreuil (FR)
(74) Mandataire: Plasseraud IP
(86) Numéro de dépôt international: PCT/FR2017/053454
(87) Numéro de publication internationale: WO 2018/115627

(56) Documents cités:
- WO-A1-2012/153073
- WO-A1-2013/018090
- WO-A1-2015/100482
- US-A1- 2007 041 600

## Description

La présente invention concerne les systèmes de substitution sensorielle, et en particulier ceux qui fournissent des stimuli tactiles pour présenter des informations visuelles à des utilisateurs, notamment malvoyants.

### ARRIERE-PLAN

Les neuroprothèses rétiniennes sont des systèmes stimulant le cerveau afin de combler le manque d'information visuelle chez des personnes souffrant d'une perte de la vision.

Ces neuroprothèses sont posées de manière invasive et stimulent électriquement des cellules rétiniennes en amont du nerf optique. Les opérations pour les mettre en place puis les utiliser sont coûteuses et peuvent mettre la santé des porteurs en danger, en raison de la présence nécessaire d'un corps étranger.

Les solutions par mutation génétique pour rendre des cellules rétiniennes photosensibles sont elles aussi invasives puisque une fois la mutation opérée, il est impossible de revenir en arrière.

D'autre part, de tels dispositifs ne sont fonctionnels que si le nerf optique du porteur est intact. Ils ne permettent donc d'aider ni des malades souffrant de glaucome ni des non-voyants de naissance. Enfin, nous ne sommes actuellement qu'aux débuts de la stimulation corticale et la recherche aura encore besoin de temps pour perfectionner ce type d'application qui sera dans tous les cas invasive. Ces problématiques touchent aussi les différentes neuroprothèses implantables telles qu'une oreille artificielle ou un capteur tactile pour les personnes avec perte de sensibilité et/ou amputées.

Pour pallier ce problème, Bach y Rita a imaginé un dispositif qui stimule les récepteurs naturels de la peau (P. Bach-y-Rita : "Tactile sensory substitution studies", Annals of the New York Academy of Sciences, n° 1013, pp. 83-91).

Des dispositifs de substitution sensorielle de ce type ont fait leurs preuves en permettant de recouvrer une partie de l'autonomie des patients. Voir D. Moraru, C.A. Boiangiu: "About Visual Sensory Substitution", Proceedings of the 3rd International Conférence on Acoustics, Speech and Audio Processing (ASAP' 15), Salerno, Italy, juin 2015, pp. 115-124, ou C.C. Pack, S.J. Bensmaia: "Seeing and Feeling Motion: Canonical Computations in Vision and Touch", PLoS Biology, Vol. 13, No. 9: e1002271, septembre 2015.

Cependant, les dispositifs de substitution sensorielle connus ne répondent pas bien aux besoins des non-voyants. Ils sont généralement encombrants et fortement consommateurs d'énergie, ce qui réduit le bénéfice tiré de leur utilisation. Voir P. Galambos: "Vibrotactile Feedback for Haptics and Telemanipulation: Survey, Concept and Experiment", Acta Polytechnica Hungarica, Vol. 9, No. 1 (2012), pp. 41-65, ou S. Maidenbaum, S. Abboud, A. Amedi: "Sensory substitution: Closing the gap between basic research and widespread practical visual réhabilitation", Neuroscience & Biobehavioral Reviews, Vol. 41 (2014), pp. 3-15. De plus, ils nécessitent un apprentissage très long et sont d'une efficacité très dépendante de la personne.

Des exemples de réalisations antérieures sont décrits dans WO 2012/153073 A1, US 2007/041600 A1, WO 2013/018090 A1 et WO 2015/100482 A1.

Un but de la présente invention est de proposer une autre technique capable de surmonter au moins en partie les difficultés ci-dessus.

### RESUME

Il est proposé un système de substitution sensorielle, comprenant : une matrice d'excitateurs, portable par un utilisateur de façon à ce que les excitateurs interagissent localement avec la peau; un circuit de commande des excitateurs de la matrice ; et une source de signal asynchrone pour fournir au circuit de commande un signal asynchrone représentatif de l'information visuelle organisée selon une matrice de pixels. Le signal asynchrone comprend, pour chaque pixel, des événements successifs associés de manière asynchrone audit pixel.

Un tel système de substitution sensorielle prend avantageusement en compte le fonctionnement du cerveau humain et des cellules de la peau. Le corps humain est capable de recevoir des informations asynchrones à l'échelle de la milliseconde. Cela lui permet de traiter l'information de manière optimale, comme l'ont montré M. J. Berry, D.K. Warland, M. Meister: "The structure and précision of retinal spike trains", Proceedings of the National Academy of Sciences, Vol. 94, No. 10 (1997), pp. 5411-5416, et P. Reinagel, R.C. Reid: "Temporal Coding of Visual Information in the Thalamus", The Journal of Neuroscience, Vol. 20, No. 14 (2000), pp. 5392-5400.

Grâce à des sources de signaux asynchrones telles que des caméras neuromorphiques, il est possible de mimer le fonctionnement de l'oeil tout en réduisant très fortement le traitement d'information nécessaire.

Cela permet une plus faible consommation du système de substitution sensorielle par voie tactile et d'effectuer un bio-mimétisme du traitement visuel.

Le système proposé permet de pallier les principaux inconvénients des systèmes de substitution sensorielle en termes d'ergonomie, d'efficacité énergétique et d'adaptabilité au fonctionnement cellulaire.

Dans une réalisation, la source de signal asynchrone comprend une caméra neuromorphique incluant des éléments photosensibles disposés suivant une matrice de pixels en regard d'une scène, le signal asynchrone comprenant, pour chaque pixel, des événements successifs provenant de manière asynchrone dudit pixel.

La caméra neuromorphique peut notamment être de type DVS ("Dynamic Vision Sensor") ou ATIS ("Asynchronous, Time-based Image Sensor").

Dans une autre réalisation, la source de signal asynchrone comprend un synthétiseur délivrant un signal ayant une représentation adresse-événement (AER).

La source de signal asynchrone peut encore comporter une mémoire où est lu le signal asynchrone fourni au circuit de commande.

Selon un mode de réalisation, la matrice d'excitateurs est supportée par un vêtement portable par l'utilisateur. Ce vêtement inclut par exemple un gilet. Il peut être en un matériau présentant une élasticité, comprenant par exemple du néoprène.

Dans une réalisation du système de substitution sensorielle, les excitateurs de la matrice comprennent des actionneurs mécaniques.

En variante, il est aussi possible que certains au moins des excitateurs de la matrice soient agencés pour interagir localement avec la peau de l'utilisateur par une stimulation parmi : stimulation électrique; variation de chaleur; injection d'une substance ; et application d'un flux d'air.

### BREVE DESCRIPTION DES DESSINS

D'autres particularités et avantages de la présente invention apparaîtront dans la description ci-après d'exemples de réalisation non limitatifs, en référence aux dessins annexés, dans lesquels :
- la figure 1 est un schéma synoptique d'un système de substitution sensorielle selon un mode de réalisation de l'invention ;
- la figure 2A est un diagramme montrant un exemple de profil d'intensité lumineuse au niveau d'un pixel d'une caméra asynchrone ;
- la figure 2B montre un exemple de signal délivré par la caméra asynchrone en réponse au profil intensité de la figure 2A ;
- la figure 2C illustre la reconstruction du profil intensité à partir du signal de la figure 2B ;
- les figures 3A-B sont des diagrammes analogues à ceux des figures 2A-B illustrant un mode d'acquisition lumineuse utilisable dans un autre exemple de réalisation du procédé ;
- la figure 4 est un schéma synoptique d'une caméra asynchrone de type ATIS ;
- la figure 5 est une vue schématique d'une matrice d'excitateurs utilisable dans certains modes de réalisation de l'invention ; et
- la figure 6 montre un vêtement équipé d'une telle matrice d'excitateurs.

### DESCRIPTION DE MODES DE REALISATION

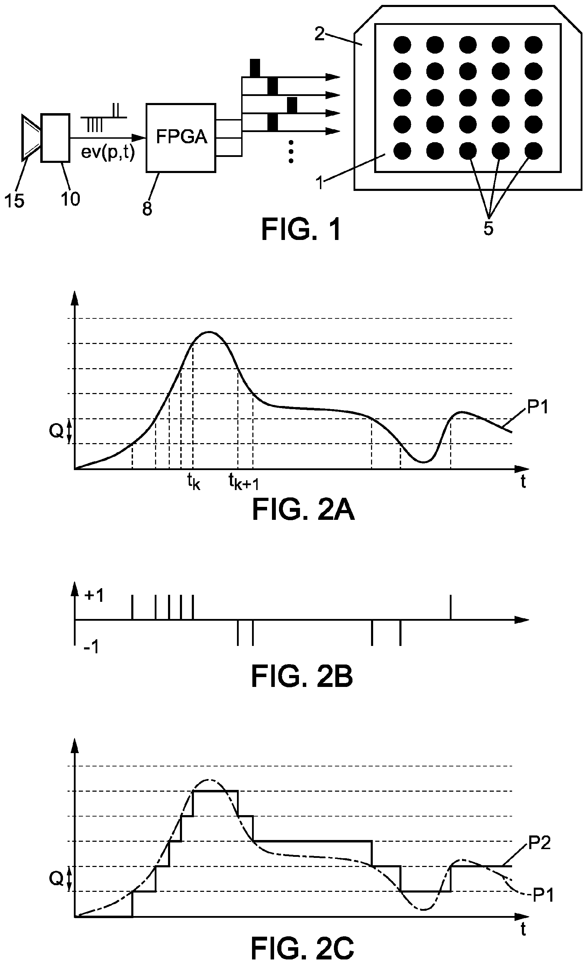

En référence à la figure 1, un système de substitution sensorielle comprend une matrice d'excitateurs 1 destinée à être placée contre la peau 2 d'un utilisateur.

Chaque excitateur 5 de la matrice 1 est placé de façon à interagir localement avec la peau de l'utilisateur lorsqu'il est activé par un circuit de commande 8.

Dans l'exemple représenté, le circuit de commande 8 est réalisé en technologie FPGA ("Field-Programmable Gate Array"). D'autres technologies sont utilisables pour réaliser le circuit de commande 8, par exemple les technologies de circuits imprimés d'application spécifique (ASIC, "Application-Specific Integrated Circuit"). Le circuit 8 produit les signaux de commande respectifs des excitateurs 5 en fonction d'un signal asynchrone ev(p, t) reçu d'une source 10.

Le signal asynchrone ev(p, t) fourni au circuit de commande 8 par la source 10 est représentatif d'information visuelle organisée selon une matrice de pixels. Il se compose d'un flux d'événements ayant chacun une adresse correspondant à un pixel p de la matrice et un temps d'occurrence t de l'événement.

À titre d'exemple, la source de signal asynchrone 10 peut être une caméra neuromorphique du genre de celles décrites dans l'article de C. Posch : "Bio-inspired vision", Journal of Instrumentation, Vol. 7, No. 1 (2012), C01054.

La figure 1 montre une telle caméra neuromorphique 10, constituant un capteur de vision asynchrone basé sur événement, placée en regard d'une scène et recevant le flux lumineux de la scène à travers une optique d'acquisition 15 comprenant une ou plusieurs lentilles. La caméra 10 est placée dans le plan image de l'optique d'acquisition 15. Elle comporte un groupement d'éléments photosensibles organisés en une matrice de pixels. Chaque pixel correspondant à un élément photosensible produit des événements successifs dépendant des variations de lumière dans la scène.

L'information visuelle asynchrone issue de la caméra 10 consiste en des séquences d'événements ev(p, t) reçus de manière asynchrone des différents pixels p en fonction des variations de lumière ressenties par le pixel dans la scène apparaissant dans le champ de vision de la caméra.

La caméra asynchrone 10 réalise une acquisition par exemple selon le principe illustré par les figures 2A-C. L'information délivrée comprend une succession d'instants tₖ (k = 0, 1, 2, ...) auxquels un seuil d'activation Q est atteint. La figure 2A montre un exemple de profil d'intensité lumineuse P1 vue par un pixel de la matrice. Chaque fois que cette intensité augmente d'une quantité égale au seuil d'activation Q à partir de ce qu'elle était au temps tₖ, un nouvel instant tₖ₊₁ est identifié et une raie positive (niveau +1 sur la figure 2B) est émise à cet instant tₖ₊₁. Symétriquement, chaque fois que l'intensité du pixel diminue de la quantité Q à partir de ce qu'elle était au temps tₖ, un nouvel instant tₖ₊₁ est identifié et une raie négative (niveau -1 sur la figure 2B) est émise à cet instant tₖ₊₁. La séquence de signal asynchrone pour le pixel consiste alors en une succession d'impulsions ou raies ("spikes") positives ou négatives positionnées dans le temps aux instants tₖ dépendant du profil lumineux pour le pixel. Ces raies peuvent être représentées mathématiquement par des pics de Dirac positifs ou négatifs et caractérisées chacune par un instant d'émission tₖ et un bit de signe. La sortie de la caméra 10 est alors sous la forme d'une représentation adresse-événement (AER, "Address-Event Représentation"). La figure 2C montre le profil d'intensité P2 qu'on est capable de reconstruire comme une approximation du profil P1 par intégration dans le temps du signal asynchrone de la figure 2B.

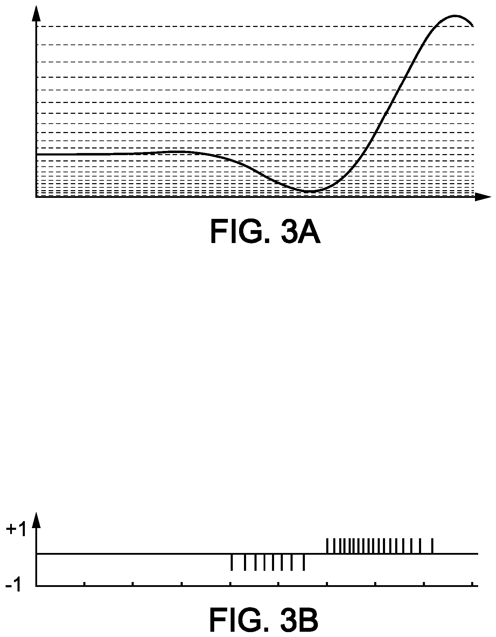

Le seuil d'activation Q peut être fixe, comme dans le cas des figures 2A-C, ou adaptatif en fonction de l'intensité lumineuse, comme dans le cas des figures 3A-B. Par exemple, le seuil ±Q peut être comparé aux variations du logarithme de l'intensité lumineuse pour la génération d'un événement ±1.

À titre d'exemple, la caméra asynchrone 10 peut être un capteur de vision dynamique (DVS) du genre décrit dans *"*A 128×128 120 dB 15 µs Latency Asynchronous Temporal Contrast Vision Sensor", P. Lichtsteiner, et al., IEEE Journal of Solid-State Circuits, Vol. 43, No. 2, février 2008, pp. 566-576, ou dans la demande de brevet US 2008/0135731 A1. La dynamique d'une rétine (durée minimum entre les potentiels d'action) de l'ordre de quelques millisecondes peut être approchée avec un DVS de ce type. La performance en dynamique est en tout cas largement supérieure à celle qu'on peut atteindre avec une caméra vidéo classique ayant une fréquence d'échantillonnage réaliste. Il est à noter que la forme du signal asynchrone délivré pour un pixel par le DVS 10, qui constitue le signal d'entrée du circuit de commande 8, peut être différente d'une succession de pics de Dirac, les événements représentés pouvant avoir une largeur temporelle ou une amplitude ou une forme d'onde quelconque dans ce signal asynchrone basé sur événement.

Un autre exemple de caméra asynchrone avantageusement utilisable dans le contexte de la présente invention est le capteur d'image asynchrone à base temporelle (ATIS) dont une description est donnée dans l'article *"*A QVGA 143 dB Dynamic Range Frame-Free PWM Image Sensor With Lossless Pixel-Level Video Compression and Time-Domain CDS", C. Posch, et al., IEEE Journal of Solid-State Circuits, Vol. 46, No. 1, janvier 2011, pp. 259-275.

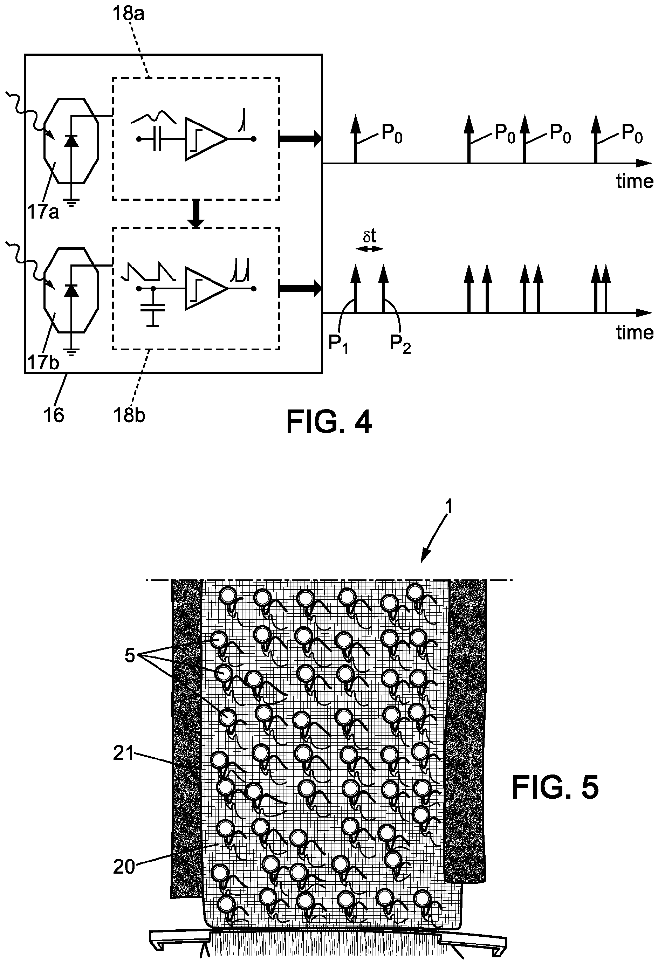

La figure 4 illustre le principe de l'ATIS. Un pixel 16 de la matrice constituant la caméra comporte deux éléments photosensibles 17a, 17b, tels que des photodiodes, respectivement associés à des circuits électroniques de détection 18a, 18b. Le capteur 17a et son circuit 18a ont un fonctionnement similaire à ceux du DVS précédemment mentionné. Ils produisent une impulsion P₀ lorsque l'intensité lumineuse reçue par la photodiode 17a varie d'une quantité prédéfinie. L'impulsion P₀ marquant ce changement d'intensité déclenche le circuit électronique 18b associé à l'autre photodiode 17b. Ce circuit 18b génère alors une première impulsion P₁ puis une deuxième impulsion P₂ dès qu'une quantité de lumière donnée (nombre de photons) est reçue par la photodiode 17b. L'écart temporel δt entre les impulsions P₁ et P₂ est inversement proportionnel à l'intensité lumineuse reçue par le pixel 16 juste après l'apparition de l'impulsion P₀. L'information asynchrone issue de l'ATIS est une autre forme de représentation AER, comprenant deux trains impulsions pour chaque pixel: le premier train des impulsions P₀ indique les instants où l'intensité lumineuse a changé au-delà du seuil de détection, tandis que le second train se compose des impulsions P₁ et P₂ dont l'écart temporel δt indique les intensités lumineuses, ou niveaux de gris, correspondants. Un événement ev(p, t) issu d'un pixel 16 de position p dans la matrice de l'ATIS comporte alors deux types d'information: une information temporelle donnée par la position de l'impulsion P₀, donnant l'instant t de l'événement, et une information de niveau de gris donnée par l'écart temporel δt entre les impulsions P₁ et P₂.

Dans une autre réalisation, la source de signal asynchrone 10 qui alimente le circuit de commande 8 n'est pas une caméra observant une scène réelle, mais un synthétiseur de signal ayant une représentation AER. Le signal asynchrone ev(p, t) est synthétisé par une telle source de manière à émuler des images à présenter à l'utilisateur, qui peuvent être plus ou moins réalistes, voire abstraites.

Par exemple, les images ainsi présentées par le système de substitution sensorielle peuvent correspondre à des éléments d'une interface utilisateur graphique (GUI) du type de celles couramment utilisées dans divers appareils tels que smartphones, tablettes, ordinateurs, téléviseurs... Le signal asynchrone produit par le synthétiseur peut résulter d'une conversion d'éléments graphiques de format classique, ou être généré directement pour les besoins du système de substitution sensorielle.

La source de signal asynchrone 10 peut également procéder par lecture dans une mémoire, de type électronique, magnétique ou optique, pour fournir au circuit de commande 8 un signal asynchrone basé sur événement qui a été préalablement enregistré après avoir été acquis soit à l'aide d'une caméra neuromorphique, soit à l'aide d'un synthétiseur.

Le circuit de commande 8 alimente les excitateurs 5 de la matrice 1 à partir du signal asynchrone reçu de la source 10. Chaque excitateur 5 reçoit une commande individuelle sous forme de tension d'alimentation fournie sélectivement lorsque des événements sont reçus pour des pixels ayant des positions correspondantes dans la représentation AER.

Chaque excitateur 5 de la matrice 1 peut consister en un actionneur mécanique, par exemple de type piézoélectrique, qui engendre des vibrations et/ou des pressions sur la peau 2 lorsqu'il est activé par son signal respectif provenant du circuit de commande 8.

D'autres types de stimulation peuvent également être appliqués localement par un excitateur 5 de la matrice 1 :
- stimulation électrique à l'aide d'électrodes alimentées sélectivement en tension par le circuit de commande 8 ;
- variation de chaleur à l'aide d'un dispositif thermique ;
- injection d'une substance ;
- application d'un flux d'air, etc.

Une combinaison d'excitateurs 5 de types différents au sein de la matrice 1 est également envisageable. Ce qui importe est le caractère local de l'excitation appliquée par chaque élément 5 de la matrice 1. Cette matrice permet ainsi de fournir une sorte de cartographie des points d'excitation sur la peau de l'utilisateur, qui produit un schéma perceptif spatialisé permettant la substitution sensorielle.

Il est possible que la densité spatiale des excitateurs 5 dans la matrice 1 soit plus faible que celle des pixels ayant servi à l'acquisition du signal asynchrone. Dans ce cas, le circuit de commande 8 peut réaliser une intégration spatiale pour engendrer les signaux de commande des excitateurs 5.

On peut également interfacer n'importe quel système générant des informations de manière asynchrone, même non visuelles. Par exemple, on peut prévoir l'utilisation d'un capteur de pression asynchrone pour les personnes amputées, ou encore un dispositif audio asynchrone. L'utilisation des dispositifs de substitution sensorielle peuvent donc être généralisés à n'importe quel capteur permettant de fournir une information sous forme de flux événementiels.

Pour simplifier la connectique des excitateurs 5, il est possible d'agencer la matrice 1 de façon qu'elle présente un plan de masse commun pour l'ensemble des excitateurs ou une partie d'entre eux. Il suffit alors d'amener la tension de commande de chaque excitateur 5 par l'intermédiaire d'un fil ou d'une piste conductrice. Le plan de masse peut notamment prendre la forme d'une nappe textile conductrice 20 connectée à une borne de l'alimentation électrique associée au circuit de commande 8, et sur laquelle sont fixés les excitateurs 5.

La figure 5 montre une telle nappe textile 20 sur laquelle les excitateurs 5, constitués dans cet exemple par des actionneurs mécaniques 5, sont installés. Dans cet exemple, la nappe 20 est cousue ou collée sur un autre support textile 21 destiné à venir au contact de la peau 2 de l'utilisateur pour lui communiquer les pressions ou les vibrations engendrées par les actionneurs 5.

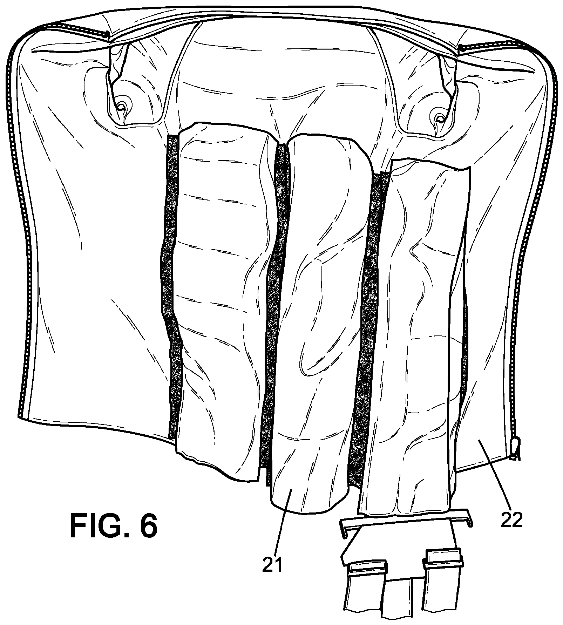

Comme le montre la figure 6, l'ensemble comprenant la matrice d'actionneurs 1 et le support 21 peut être fixé à l'intérieur d'un vêtement 22 portable par l'utilisateur de façon que le support 21 soit contre la peau de l'utilisateur afin de réaliser le couplage mécanique désiré avec les actionneurs 5.

Le vêtement 22 est de préférence réalisé en un matériau présentant une élasticité pour bien appliquer la matrice d'actionneurs 1 contre la peau 2 de l'utilisateur. À titre d'exemple, le vêtement 22 peut être réalisé en néoprène. Typiquement, il pourra s'agir d'un gilet, de telle sorte que la matrice d'actionneurs 1 soit placée contre le dos de l'utilisateur, éventuellement ses flancs, et éventuellement son ventre. Comme le montre la figure 6, les actionneurs 5 peuvent être distribués en plusieurs sous-ensembles pour limiter la rigidité globale du vêtement équipé.

Bien entendu, la matrice 1 peut comporter des excitateurs 5 distribués sur d'autres parties du corps de l'utilisateur, notamment les membres. Le vêtement qui la supporte peut être une combinaison couvrant plus que le buste de l'utilisateur.

Le système de substitution sensorielle décrit ci-dessus prend en compte le fonctionnement des cellules réceptrices de la peau du point de vue de l'interprétation spatiotemporelle faite par le cerveau. Ainsi, il est en mesure de générer des stimuli tactiles, électriques, physiques, ou autres, de manière asynchrone en différents endroits du corps.

Les excitateurs 5 sont répartis de manière à se conformer à la densité de cellules réceptrices de la zone stimulée.

Les informations visuelles communiquées avec la substitution sensorielle peuvent être espacées de quelques microsecondes. Les tensions d'alimentation des excitateurs 5 présentent des impulsions qui durent le temps nécessaire pour stimuler les cellules réceptrices avec l'intensité souhaitée. Un grand nombre de stratégies de stimulation peuvent être mis en place en fonction de la nature de l'information à transmettre. On peut notamment simuler les différents niveaux de gris d'une image, ou encore simuler l'écriture d'un texte pour la reconnaissance de mots.

Des tests ont été réalisés sur des personnes saines afin d'étudier leurs réponses à de tels stimuli. Ces tests, réalisés avec des actionneurs mécaniques 5 dans la matrice 1, ont fourni des résultats très encourageants. Après quelques minutes d'entraînement, la reconnaissance de lettres et de chiffres, ainsi que de diverses formes géométriques, était déjà satisfaisante. Ces tests ont également permis d'identifier le déplacement d'objets à grande vitesse dans le champ visuel d'une caméra neuromorphique 10 telle que décrite ci-dessus. C'est un résultat très prometteur en vue d'autres expériences dynamiques de mobilité. L'idiosyncrasie des personnes peut également être prise en compte afin d'être capable d'interfacer le système avec chaque sujet.

Les modes de réalisation décrits ci-dessus sont une simple illustration de la présente invention. Diverses modifications peuvent leur être apportées sans sortir du cadre de l'invention qui ressort des revendications annexées.

## Revendications

1. Système de substitution sensorielle, comprenant :
une matrice d'excitateurs (1) portable par un utilisateur de façon que les excitateurs (5) interagissent localement avec la peau (2) de l'utilisateur ;
un circuit de commande (8) des excitateurs de la matrice ; **caractérisé en ce qu'**il comprend en outre
une source de signal asynchrone (10) pour fournir au circuit de commande un signal asynchrone représentatif d'information visuelle organisée selon une matrice de pixels, le signal asynchrone comprenant, pour chaque pixel, des événements successifs associés de manière asynchrone audit pixel.

2. Système selon la revendication 1, dans lequel la source de signal asynchrone (10) comprend une caméra neuromorphique incluant des éléments photosensibles disposés suivant la matrice de pixels en regard d'une scène, le signal asynchrone comprenant, pour chaque pixel, des événements successifs provenant de manière asynchrone dudit pixel.

3. Système selon la revendication 2, dans lequel la caméra neuromorphique (10) est de type DVS ("Dynamic Vision Sensor") ou ATIS ("Asynchronous, Time-based Image Sensor").

4. Système selon la revendication 1, dans lequel la source de signal asynchrone comprend un synthétiseur délivrant un signal ayant une représentation adresse-événement (AER).

5. Système selon la revendication 1, dans lequel la source de signal asynchrone comprend une mémoire où est lu le signal asynchrone fourni au circuit de commande (8)

6. Système selon l'une quelconque des revendications précédentes, dans lequel la matrice d'excitateurs (1) est supportée par un vêtement (22) portable par l'utilisateur.

7. Système selon la revendication 6, dans lequel le vêtement (22) inclut un gilet.

8. Système selon la revendication 6 ou la revendication 7, dans lequel le vêtement (22) est en un matériau présentant une élasticité

9. Système selon la revendication 8, dans lequel le matériau présentant une élasticité comprend du néoprène.

10. Système selon l'une quelconque des revendications précédentes, dans lequel les excitateurs (5) de la matrice (1) comprennent des actionneurs mécaniques.

11. Système selon l'une quelconque des revendications précédentes, dans lequel certains au moins des excitateurs (5) de la matrice (1) sont agencés pour interagir localement avec la peau (2) de l'utilisateur par une stimulation parmi : stimulation électrique ; variation de chaleur ; injection d'une substance ; et application d'un flux d'air.

## Patentansprüche

1. System zur sensorischen Substitution, umfassend:
eine Erregermatrix (1), die von einem Benutzer getragen werden kann, so dass die Erreger (5) lokal mit der Haut (2) des Benutzers interagieren;
eine Steuerschaltung (8) für die Matrixerreger; **dadurch gekennzeichnet, dass** sie ferner eine asynchrone Signalquelle (10) umfasst, um der Steuerschaltung ein asynchrones Signal zu liefern, das für visuelle Informationen repräsentativ ist, die in einer Matrix von Pixeln organisiert sind, wobei das asynchrone Signal für jedes Pixel aufeinanderfolgende Ereignisse umfasst, die asynchron mit dem Pixel verknüpft sind.

2. System nach Anspruch 1, wobei die asynchrone Signalquelle (10) eine neuromorphe Kamera mit lichtempfindlichen Elementen umfasst, die entlang der Pixelmatrix gegenüber einer Szene angeordnet sind, wobei das asynchrone Signal für jedes Pixel aufeinanderfolgende Ereignisse umfasst, die asynchron von dem Pixel stammen.

3. System nach Anspruch 2, wobei die neuromorphe Kamera (10) vom Typ DVS ("Dynamic Vision Sensor") oder ATIS ("Asynchronous, Time-based Image Sensor") ist.

4. System nach Anspruch 1, wobei die asynchrone Signalquelle einen Synthesizer umfasst, der ein Signal mit einer Adresse-Ereignis-Darstellung (AER) ausgibt.

5. System nach Anspruch 1, wobei die asynchrone Signalquelle einen Speicher umfasst, aus dem das an die Steuerschaltung (8) gelieferte asynchrone Signal ausgelesen wird.

6. System nach einem der vorhergehenden Ansprüche, wobei die Erregermatrix (1) an einem vom Benutzer tragbaren Kleidungsstück (22) getragen ist.

7. System nach Anspruch 6, wobei das Kleidungsstück (22) eine Weste umfasst.

8. System nach Anspruch 6 oder Anspruch 7, wobei das Kleidungsstück (22) aus einem Material mit Elastizität besteht.

9. System nach Anspruch 8, wobei das Material mit Elastizität Neopren umfasst.

10. System nach einem der vorhergehenden Ansprüche, wobei die Erreger (5) der Matrix (1) mechanische Aktuatoren umfassen.

11. System nach einem der vorhergehenden Ansprüche, wobei wenigstens einige der Erreger (5) der Matrix (1) so angeordnet sind, dass sie lokal mit der Haut (2) des Benutzers durch eine der folgenden Stimulationen interagieren: elektrische Stimulation; Wärmeveränderung; Injektion einer Substanz; und Anwendung eines Luftstroms.

## Claims

1. A sensory substitution system, comprising:
a matrix of exciters (1) that can be worn by a user in such a way that the exciters (5) interact locally with the user's skin (2),
a control circuit (8) for the exciters of the matrix,
**characterized in that** it further comprises
an asynchronous signal source (10) for providing the control circuit with an asynchronous signal representative of visual information organised according to a matrix of pixels, with the asynchronous signal comprising, for each pixel, successive events associated in an asynchronous manner with said pixel.

2. A system as claimed in claim 1, wherein the asynchronous signal source (10) comprises a neuromorphic camera including photosensitive elements arranged according to the matrix of pixels facing a scene, with the asynchronous signal comprising, for each pixel, successive events originating in an asynchronous manner from said pixel.

3. A system as claimed in claim 2, wherein the neuromorphic camera (10) is of a DVS ("Dynamic Vision Sensor") or ATIS ("Asynchronous Time-based Image Sensor") type.

4. A system as claimed in claim 1, wherein the asynchronous signal source comprises a synthesizer producing a signal with an address-event representation (AER).

5. A system as claimed in claim 1, wherein the asynchronous signal source comprises a memory where the asynchronous signal supplied to the control circuit (8) is read.

6. A system as claimed in any one of the preceding claims, wherein the matrix of exciters (1) is attached to a garment (22) that can be worn by the user.

7. A system as claimed in claim 6, wherein the garment (22) includes a vest.

8. A system as claimed in claim 6 or claim 7, wherein the garment (22) is made from a material having elasticity

9. A system as claimed in claim 8, wherein the material having elasticity comprises neoprene.

10. A system as claimed in any one of the preceding claims, wherein the exciters (5) of the matrix (1) comprise mechanical actuators.

11. A system as claimed in any one of the preceding claims, wherein at least some of the exciters (5) of the matrix (1) are arranged to interact locally with the user's skin (2) through stimulation, including electrical stimulation, heat variation, injection of a substance and application of an air flow.
